# EUROPÄISCHE PATENTSCHRIFT

(11) **EP 2 437 748 B1**
(45) Veröffentlichungstag und Bekanntmachung des Hinweises auf die Patenterteilung: **31.08.2016**
(21) Anmeldenummer: 10800714.7
(22) Anmeldetag: 10.12.2010
(51) Int. Cl.: A61K 31/573, A61K 38/17, A61K 38/20, A61K 45/06, A61P 19/02, A61P 29/00, A61P 37/00, A61K 38/00, A61K 9/00, A61K 35/15, A61K 35/19, A61K 9/51

(54) **KOMBINATIONSPRÄPARATE MIT CYTOKIN-ANTAGONIST UND CORTICOSTEROID**
COMBINATION PREPARATION COMPRISING A CYTOKINE ANTAGONIST AND A CORTICOSTEROID
PRÉPARATION COMBINÉE CONTENANT UN ANTAGONISTE DE LA CYTOKINE ET UN CORTICOSTÉROÏDE

(30) Priorität: 10.12.2009 DE 102009057495
(43) Veröffentlichungstag der Anmeldung: 11.04.2012
(73) Patentinhaber: Orthogen AG, 40210 Düsseldorf (DE)
(72) Erfinder: WEHLING, Peter, 40597 Düsseldorf (DE); REINECKE, Julio, 50733 Köln (DE)
(74) Vertreter: König, Gregor Sebastian
(86) Internationale Anmeldenummer: PCT/EP2010/069427
(87) Internationale Veröffentlichungsnummer: WO 2011/082951

(56) Entgegenhaltungen:
- WO-A2-2006/007529
- BRESNIHAN BARRY ET AL: "Treatment of rheumatoid arthritis with recombinant human interleukin-1 receptor antagonist", ARTHRITIS AND RHEUMATISM, Bd. 41, Nr. 12, Dezember 1998 (1998-12), Seiten 2196-2204, XP009146178, ISSN: 0004-3591
- SETTAS LUCAS D ET AL: "Reactivation of pulmonary tuberculosis in a patient with rheumatoid arthritis during treatment with IL-1 receptor antagonists (anakinra).", JOURNAL OF CLINICAL RHEUMATOLOGY : PRACTICAL REPORTS ON RHEUMATIC & MUSCULOSKELETAL DISEASES AUG 2007 LNKD- PUBMED:17762459, Bd. 13, Nr. 4, August 2007 (2007-08), Seiten 219-220, XP009146179, ISSN: 1076-1608
- BIANCO NICOLE R ET AL: "Therapeutic Effect of Exosomes From Indoleamine 2,3-Dioxygenase-Positive Dendritic Cells in Collagen-Induced Arthritis and Delayed-Type Hypersensitivity Disease Models", ARTHRITIS & RHEUMATISM, Bd. 60, Nr. 2, Februar 2009 (2009-02), Seiten 380-389, XP002628890, ISSN: 0004-3591
- Dr med F.-U. Hornstein: "Orthokin: "Black box" bei Arthrose?", Arznei-telegramm arznei-telegramm, Bd. 32, Nr. 3 2001, Seiten 34-34, XP002629197, Gefunden im Internet: URL:http://www.arznei-telegramm.de/registe r/0103034.pdf [gefunden am 2011-03-21]

### SEQUENZPROTOKOLL

<110> Orthogen AG
<120> Kombinationspräparate mit Cytokin-Antagonist und Corticosteroid
<130> 52 252 K
<160> 8
<170> PatentIn version 3.3
<210> 1
   <211> 177
   <212> PRT
   <213> Homo sapiens
<400> 1
   Met Glu Ile Cys Arg Gly Leu Arg Ser His Leu Ile Thr Leu Leu Leu
   1               5                   10                  15
   Phe Leu Phe His Ser Glu Thr Ile Cys Arg Pro Ser Gly Arg Lys Ser
               20                  25                  30
   Ser Lys Met Gln Ala Phe Arg Ile Trp Asp Val Asn Gln Lys Thr Phe
           35                  40                  45
   Tyr Leu Arg Asn Asn Gln Leu Val Ala Gly Tyr Leu Gln Gly Pro Asn
       50                  55                  60
   Val Asn Leu Glu Glu Lys Ile Asp Val Val Pro Ile Glu Pro His Ala
   65                  70                  75                  80
   Leu Phe Leu Gly Ile His Gly Gly Lys Met Cys Leu Ser Cys Val Lys
                   85                  90                  95
   Ser Gly Asp Glu Thr Arg Leu Gln Leu Glu Ala Val Asn Ile Thr Asp
               100                 105                 110
   Leu Ser Glu Asn Arg Lys Gln Asp Lys Arg Phe Ala Phe Ile Arg Ser
           115                 120                 125
   Asp Ser Gly Pro Thr Thr Ser Phe Glu Ser Ala Ala Cys Pro Gly Trp
       130                 135                 140
   Phe Leu Cys Thr Ala Met Glu Ala Asp Gln Pro Val Ser Leu Thr Asn
   145                 150                 155                 160
   Met Pro Asp Glu Gly Val Met Val Thr Lys Phe Tyr Phe Gln Glu Asp
                   165                 170                 175
   Glu
<210> 2
   <211> 159
   <212> PRT
   <213> Homo sapiens
<400> 2
   Met Ala Leu Glu Thr Ile Cys Arg Pro Ser Gly Arg Lys Ser Ser Lys
   1               5                   10                  15
   Met Gln Ala Phe Arg Ile Trp Asp Val Asn Gln Lys Thr Phe Tyr Leu
               20                  25                  30
   Arg Asn Asn Gln Leu Val Ala Gly Tyr Leu Gln Gly Pro Asn Val Asn
           35                  40                  45
   Leu Glu Glu Lys Ile Asp Val Val Pro Ile Glu Pro His Ala Leu Phe
       50                  55                  60
   Leu Gly Ile His Gly Gly Lys Met Cys Leu Ser Cys Val Lys Ser Gly
   65                  70                  75                  80
   Asp Glu Thr Arg Leu Gln Leu Glu Ala Val Asn Ile Thr Asp Leu Ser
                   85                  90                  95
   Glu Asn Arg Lys Gln Asp Lys Arg Phe Ala Phe Ile Arg Ser Asp Ser
               100                 105                 110
   Gly Pro Thr Thr Ser Phe Glu Ser Ala Ala Cys Pro Gly Trp Phe Leu
           115                 120                 125
   Cys Thr Ala Met Glu Ala Asp Gln Pro Val Ser Leu Thr Asn Met Pro
       130                 135                 140
   Asp Glu Gly Val Met Val Thr Lys Phe Tyr Phe Gln Glu Asp Glu
   145                 150                 155
<210> 3
   <211> 180
   <212> PRT
   <213> Homo sapiens
<400> 3
   Met Ala Leu Ala Asp Leu Tyr Glu Glu Gly Gly Gly Gly Gly Gly Glu
   1               5                   10                  15
   Gly Glu Asp Asn Ala Asp Ser Lys Glu Thr Ile Cys Arg Pro Ser Gly
               20                  25                  30
   Arg Lys Ser Ser Lys Met Gln Ala Phe Arg Ile Trp Asp Val Asn Gln
           35                  40                  45
   Lys Thr Phe Tyr Leu Arg Asn Asn Gln Leu Val Ala Gly Tyr Leu Gln
       50                  55                  60
   Gly Pro Asn Val Asn Leu Glu Glu Lys Ile Asp Val Val Pro Ile Glu
   65                  70                  75                  80
   Pro His Ala Leu Phe Leu Gly Ile His Gly Gly Lys Met Cys Leu Ser
                   85                  90                  95
   Cys Val Lys Ser Gly Asp Glu Thr Arg Leu Gln Leu Glu Ala Val Asn
               100                 105                 110
   Ile Thr Asp Leu Ser Glu Asn Arg Lys Gln Asp Lys Arg Phe Ala Phe
           115                 120                 125
   Ile Arg Ser Asp Ser Gly Pro Thr Thr Ser Phe Glu Ser Ala Ala Cys
       130                 135                 140
   Pro Gly Trp Phe Leu Cys Thr Ala Met Glu Ala Asp Gln Pro Val Ser
   145                 150                 155                 160
   Leu Thr Asn Met Pro Asp Glu Gly Val Met Val Thr Lys Phe Tyr Phe
                   165                 170                 175
   Gln Glu Asp Glu
               180
<210> 4
   <211> 143
   <212> PRT
   <213> Homo sapiens
<400> 4
   Met Gln Ala Phe Arg Ile Trp Asp Val Asn Gln Lys Thr Phe Tyr Leu
   1               5                   10                  15
   Arg Asn Asn Gln Leu Val Ala Gly Tyr Leu Gln Gly Pro Asn Val Asn
               20                  25                  30
   Leu Glu Glu Lys Ile Asp Val Val Pro Ile Glu Pro His Ala Leu Phe
           35                  40                  45
   Leu Gly Ile His Gly Gly Lys Met Cys Leu Ser Cys Val Lys Ser Gly
       50                  55                  60
   Asp Glu Thr Arg Leu Gln Leu Glu Ala Val Asn Ile Thr Asp Leu Ser
   65                  70                  75                  80
   Glu Asn Arg Lys Gln Asp Lys Arg Phe Ala Phe Ile Arg Ser Asp Ser
                   85                  90                  95
   Gly Pro Thr Thr Ser Phe Glu Ser Ala Ala Cys Pro Gly Trp Phe Leu
               100                 105                 110
   Cys Thr Ala Met Glu Ala Asp Gln Pro Val Ser Leu Thr Asn Met Pro
           115                 120                 125
   Asp Glu Gly Val Met Val Thr Lys Phe Tyr Phe Gln Glu Asp Glu
       130                 135                 140
<210> 5
   <211> 155
   <212> PRT
   <213> Homo sapiens
<400> 5
<210> 6
   <211> 177
   <212> PRT
   <213> Equus caballus
<400> 6
   Met Glu Ile Arg Arg Arg Ser Val Arg His Leu Ile Ser Leu Leu Leu
   1               5                   10                  15
   Phe Leu Phe Tyr Ser Glu Thr Ala Cys His Pro Leu Gly Lys Arg Pro
               20                  25                  30
   Cys Lys Met Gln Ala Phe Arg Ile Trp Asp Val Asn Gln Lys Thr Phe
           35                  40                  45
   Tyr Met Arg Asn Asn Gln Leu Val Ala Gly Tyr Leu Gln Glu Ser Asn
   50 55 60
   Thr Lys Leu Gln Glu Lys Ile Asp Val Val Pro Ile Glu Pro Asp Ala
   65 70 75 80
   Leu Phe Leu Gly Leu His Gly Arg Lys Leu Cys Leu Ala Cys Val Lys
   85 90 95
   Ser Gly Asp Glu Ile Arg Phe Gln Leu Glu Ala Val Asn Ile Thr Asp
   100 105 110
   Leu Ser Lys Asn Lys Glu Glu Asn Lys Arg Phe Thr Phe Ile Arg Ser
   115 120 125
   Asn Ser Gly Pro Thr Thr Ser Phe Glu Ser Ala Ala Cys Pro Gly Trp
   130 135 140
   Phe Leu Cys Thr Ala Gln Glu Ala Asp Arg Pro Val Ser Leu Thr Asn
   145 150 155 160
   Lys Pro Lys Glu Ser Phe Met Val Thr Lys Phe Tyr Leu Gln Glu Asp
   165 170 175
   Gln
<210> 7
   <211> 177
   <212> PRT
   <213> Equus caballus
<400> 7
   Met Glu Ile Arg Arg Arg Ser Val Arg His Leu Ile Ser Leu Leu Leu
   1 5 10 15
   Phe Leu Leu Tyr Ser Glu Thr Ala Cys His Pro Leu Gly Lys Arg Pro
   20 25 30
   Cys Lys Met Gln Ala Phe Arg Ile Trp Asp Val Asn Gln Lys Thr Phe
   35 40 45
   Tyr Met Arg Asn Asn Gln Leu Val Ala Gly Tyr Leu Gln Glu Ser Asn
   50 55 60
   Thr Lys Leu Gln Glu Lys Ile Asp Val Val Pro Ile Glu Pro Asp Ala
   65 70 75 80
   Leu Phe Leu Gly Leu His Gly Arg Lys Leu Cys Leu Ala Cys Val Lys
   85 90 95
   Ser Gly Asp Glu Ile Arg Phe Gln Leu Glu Ala Val Asn Ile Thr Asp
   100 105 110
   Leu Ser Lys Asn Lys Glu Glu Asn Lys Arg Phe Thr Phe Ile Arg Ser
   115 120 125
   Asn Ser Gly Pro Thr Thr Ser Phe Glu Ser Ala Ala Cys Pro Gly Trp
   130 135 140
   Phe Leu Cys Thr Ala Gln Glu Ala Asp Arg Pro Val Ser Leu Thr Asn
   145 150 155 160
   Lys Pro Lys Glu Ser Phe Met Val Thr Lys Phe Tyr Leu Gln Glu Asp
   165 170 175
   Gln
<210> 8
   <211> 176
   <212> PRT
   <213> Canis familiaris
<400> 8
   Met Glu Thr Cys Arg Cys Pro Leu Ser Tyr Leu Ile Ser Phe Leu Leu
   1 5 10 15
   Phe Leu Ser His Ser Glu Thr Ala Cys Arg Pro Leu Gly Lys Arg Pro
   20 25 30
   Cys Arg Met Gln Ala Phe Arg Ile Trp Asp Val Asn Gln Lys Thr Phe
   35 40 45
   Tyr Leu Arg Asn Asn Gln Leu Val Ala Gly Tyr Leu Gln Gly Ser Asn
   50 55 60
   Thr Lys Leu Glu Glu Lys Leu Asp Val Val Pro Val Glu Pro His Ala
   65 70 75 80
   Val Phe Leu Gly Ile His Gly Gly Lys Leu Cys Leu Ala Cys Val Lys
   85 90 95
   Ser Gly Asp Glu Thr Arg Leu Gln Leu Glu Ala Val Asn Ile Thr Asp
   100 105 110
   Leu Ser Lys Asn Lys Asp Gln Asp Lys Arg Phe Thr Phe Ile Leu Ser
   115 120 125
   Asp Ser Gly Pro Thr Thr Ser Phe Glu Ser Ala Ala Cys Pro Gly Trp
   130 135 140
   Phe Leu Cys Thr Ala Leu Glu Ala Asp Arg Pro Val Ser Leu Thr Asn
   145 150 155 160
   Arg Pro Glu Glu Ala Met Met Val Thr Lys Phe Tyr Phe Gln Lys Glu
                   165                 170                 175

## Beschreibung

### Gebiet der Erfindung

Die vorliegende Erfindung betrifft pharmazeutische Zusammensetzungen für eine Kombinationstherapie mit einem Cytokin-Antagonisten, einem weiteren Cytokin-Antagonisten und einem Corticosteroid. Mittels der Kombinationstherapie können Krankheiten wie Arthrose (inklusive entzündlicher Formen der Arthrose), Sehnenerkrankungen und/oder degenerative Wirbelsäulenerkrankungen behandelt werden, wobei die Behandlung vorzugsweise lokal erfolgt.

### Technischer Hintergrund

Arthrose bezeichnet einen "Gelenkverschleiß", der das altersübliche Maß übersteigt. Er geht einher mit Verlust von Knorpel im betroffenen Gelenk, woraus sich Schmerzen und Funktionsverschlechterungen ergeben. Ursächlich werden ein Übermaß an Belastung, angeborene oder traumatisch bedingte Ursachen, wie Fehlstellungen der Gelenke, oder auch knöcherner Deformierung durch Knochenerkrankungen wie Osteoporose gesehen. Sie kann ebenfalls als Folge einer anderen Erkrankung wie etwa Gelenkentzündung entstehen oder mit überlastungsbedingter Ergussbildung einhergehen.

Grundsätzlich können alle Gelenke von arthrotischen Veränderungen betroffen werden. In Deutschland wird die Erkrankung am häufigsten im Kniegelenk lokalisiert. Arthrose ist einer der häufigsten Beratungsanlässe in einer allgemeinmedizinischen Praxis. Etwa 10% der Bevölkerung in westlichen Ländern leidet an Arthrose. Rechnet man die Arthrosen der kleinen Wirbelgelenke und die degenerativen Bandscheibenerkrankungen dazu, sind sogar ca. 15%-20% der Bevölkerung betroffen. Das Risiko an Arthrose zu erkranken erhöht sich mit zunehmendem Alter. Etwa zwei Drittel der Menschen über 65 Jahren sind von der Erkrankung betroffen, jedoch leiden nicht alle Betroffenen auch an den Symptomen.

Für die Behandlung von Arthrose sind bereits einige Therapieformen bekannt. Hierzu gehören sowohl konservative (z.B. medikamentöse) Therapien als auch chirurgische Eingriffe bis hin zur Ersetzung des vollständigen Gelenks durch eine Prothese. Um solche umfangreichen und unumkehrbaren Eingriffe zu vermeiden, ist grundsätzlich eine wirksame medikamentöse Behandlung vorzuziehen, um den Zeitpunkt eines kompletten Gelenkersatzes möglichst weit herauszuzögern.

Allerdings haben viele medikamentöse Behandlungen auch Nachteile. Zum einen liegt dies an den Nebenwirkungen der Medikamente selbst, aber auch ihre Wirksamkeit ist teilweise nur bedingt gegeben.

Ein oft verwendeter medizinischer Wirkstoff zur Behandlung von Arthrose ist Cortison und verwandte Corticosteroide. Diese werden systemisch allerdings meist lokal als Injektion in das betroffene Gelenk verabreicht. Hierbei zeigt sich allerdings, dass die positive Wirkung des Corticosteroids bereits nach nur einer Woche nachlässt. Dies ist klinisch aufgrund randomisierter Studien und klinischer Erfahrungen gesichert.

Ein weiteres Medikament, das zur Behandlung von Arthrose eingesetzt werden kann, ist das körpereigene Protein IL-1Ra oder eine Isoform oder ein Fragment hiervon, welches eine ähnliche Aktivität aufweist. Interleukin-1-Rezeptorantagonist (IL-1 Ra) bindet an dieselben Rezeptoren auf der Zelloberfläche wie Interleukin-1 (IL-1), löst dort aber nicht die normalerweise durch IL-1-Bindung hervorgerufene Signalkaskade aus. Durch Bindung an den IL-1-Rezeptor blockiert IL-1 Ra die Bindung von IL-1 und verhindert so dessen Signalweiterleitung und somit die entzündungsfördernde Wirkung von IL-1 auf die Zielzellen.

Die Behandlung von Patienten mit körpereigenem Serum, in dem IL-1Ra angereichert wurde und neben anderen Faktoren enthalten ist, ist im Stand der Technik bekannt. Das so eingesetzte IL-1Ra wird auch Orthokin genannt. Ein rekombinantes IL-1Ra-Fragment, Anakinra, zeigte hingegen überraschenderweise bei der Behandlung von Arthrosen keinerlei Wirkung im Vergleich zu einer Placebo-Behandlung. Anakinra ist eine auf die Aminosäuren 26 - 177 verkürzte und endständig L-methionylierte Isoform des humanen Interleukin-1-Rezeptorantagonisten und besitzt eine Sequenzlänge von 153 Aminosäuren. Die Herstellung erfolgt zum Beispiel durch *Escherichia coli*-Stämme mit rekombinanten Methoden.

WO 2006/007529 A2 offenbart verschiedene Behandlungen mit Cytokin-Antagonisten, insbesondere natürlich vorkommendem IL-1 Ra.

In der Veröffentlichung "arznei-telegramm" 2001; Jg. 32, Nr. 3, S. 34 wird der Einsatz von Orthokin bei Arthrose, rheumatoider Arthritis und Bandscheibenvorfall allgemein diskutiert.

Bianco et al. (2009) Arthritis & Rheumatism 60, 380 beschäftigt sich mit der Wirkung von Exosomen aus dendritischen Zellen auf verschiedene Mausmodelle von Krankheiten.

Bresnihan et al. (1998) Arthritis & Rheumatism 41, 2196 offenbart die Behandlung von rheumatoider Arthritis mit rekombinantem humanem IL-1Ra. Ein Teil der Patienten verwendete ein Corticosteroid (Prednisolon), ohne dass eine Wirkung dieser Maßnahme offenbart wäre.

Settas et al. (2007) offenbart die Reaktivierung einer Lungen-Tuberkulose bei einem an rheumatoider Arthritis leidenden Patienten, der mit rekombinantem IL-1Ra (Anakinra) behandelt wurde. Er nahm zusätzlich Prednisolon ein, ohne dass eine Wirkung hiervon offenbart wäre.

Angesichts des Stands der Technik gab es daher die Aufgabe, eine medikamentöse Arthrose-Behandlung bereitzustellen, die eine bessere Wirksamkeit aufweist und insbesondere eine gute Langzeit-Wirksamkeit besitzt.

### Zusammenfassung der Erfindung

Überraschenderweise wurde nun festgestellt, dass die Wirksamkeit, insbesondere die Langzeit-Wirksamkeit, von Corticosteroiden wie Cortison bei Arthrose, entzündlichen Formen der Arthrose und degenerativer Wirbelsäulenerkrankung durch die zusätzliche Verabreichung eines Cytokin-Antagonisten wie Orthokin und Anakinra deutlich bzw. synergistisch verbessert werden kann. Dies zeigt sich insbesondere bei der lokalen Verabreichung der Therapeutika direkt in das zu behandelnde Gelenk. Dies ist insbesondere äußerst überraschend angesichts der Tatsache, dass dieser vorteilhafte Effekt bei zusätzlicher Verabreichung sowohl von natürlichem IL-1Ra wie Orthokin als auch von rekombinantem IL-1 Ra wie Anakinra auftritt, obwohl Anakinra alleine erwiesenermaßen keinerlei Wirkung bei der Behandlung von Arthose und degenerativen Wirbelsäulenerkrankungen zeigt. Die vorliegende Erfindung eröffnet die Möglichkeit, das rekombinante IL-1Ra für eine Behandlung der Arthrose und Wirbelsäulenerkrankungen tauglich zu machen, da dieses in alleiniger Form nicht zur Behandlung dieser Erkrankungen geeignet ist. Eine ähnliche, überraschend gute Wirksamkeit und hohe Sicherheit der Kombination dieser Wirkstoffe wurde auch für Autoimmunkrankheiten wie Neurodermitis und kreisrunder Haarausfall gefunden, wobei vor allem die entzündungshemmende Wirkung eine Rolle spielt.

Eine mögliche Erklärung für diesen Umstand ist, dass die Cytokin-Antagonisten eine anabole Wirkung haben und die in den betroffenen Gelenken bekannte schädliche katabole Wirkung des Corticosteroids aufheben oder gar umkehren können. Daher können die Corticosteroide bei der Behandlung von zum Beispiel Arthrose neben den Cytokin-Antagonisten alternativ oder zusätzlich auch mit anabolen Wachstumsfaktoren kombiniert werden, um eine ähnliche oder sogar sich potenzierende Wirkung zu erzielen. Die Erfindung erlaubt es also, ein Corticosteroid zu entwickeln, das die bekannten schädlichen Wirkungen bei Arthrose, die aus einer Verstärkung der Knorpelzerstörung bestehen können, nicht aufweist, indem es mit einem Cytokinantagonisten kombiniert wird.

Daher stellt die vorliegende Erfindung in einem ersten Aspekt eine pharmazeutische Zusammensetzung bereit, die ein Corticosteroid zusammen mit einem Cytokin-Antagonisten, einem weiteren Cytokin-Antagonisten und optional einem Wachstumsfaktor enthält, wobei der Cytokin-Antagonist der rekombinante Interleukin-Antagonist IL-1Ra-Protein ist, insbesondere Anakinra, der weitere Cytokin-Antagonist der natürlich vorkommende Interleukin-Antagonist IL-1Ra-Protein ist, insbesondere Orthokin, und wobei die pharmazeutische Zusammensetzung für eine lokale Verabreichung geeignet ist.

Ebenso können die Therapeutika auch in zwei verschiedenen pharmazeutischen Zusammensetzungen gleichzeitig oder in zeitlicher Abfolge verabreicht werden. Entsprechend wird in einem zweiten und einem dritten Aspekt der Erfindung eine pharmazeutische Zusammensetzung enthaltend einen Cytokin-Antagonisten, einen weiteren Cytokin-Antagonisten und optional einen Wachstumsfaktor zur Verwendung in einer Kombinationstherapie zusammen mit einem Corticosteroid sowie eine pharmazeutische Zusammensetzung enthaltend ein Corticosteroid zur Verwendung in einer Kombinationstherapie zusammen mit einem Cytokin-Antagonisten, einem weiteren Cytokin-Antagonisten und optional einem Wachstumsfaktor bereitgestellt. Sowohl im Fall des zweiten als auch des dritten Aspekts der Erfindung ist der Cytokin-Antagonist der rekombinante Interleukin-Antagonist IL-1 Ra-Protein, insbesondere Anakinra und der weitere Cytokin-Antagonist der natürlich vorkommende Interleukin-Antagonist IL-1Ra-Protein, und die pharmazeutische Zusammensetzung ist für eine lokale Verabreichung geeignet.

In einem vierten erfindungsgemäßen Aspekt wird ein Kit bereitgestellt, der eine einen Cytokin-Antagonisten und optional einen Wachstumsfaktor enthaltende pharmazeutische Zusammensetzung, eine ein Corticosteroid enthaltende pharmazeutische Zusammensetzung und außerdem einen weiteren Cytokin-Antagonisten enthält. Der Cytokin-Antagonist ist der rekombinante Interleukin-Antagonist IL-1Ra-Protein, insbesondere Anakinra, der weitere Cytokin-Antagonist der natürlich vorkommende Interleukin-Antagonist IL-1Ra-Protein, und die pharmazeutischen Zusammensetzungen sind für eine lokale Verabreichung geeignet.

Außerdem betrifft die Erfindung in einem fünften Aspekt die Verwendung eines Cytokin-Antagonisten, eines weiteren Cytokin-Antagonisten und optional eines Wachstumsfaktors zur Herstellung einer pharmazeutischen Zusammensetzung zur Verwendung in einer Kombinationstherapie zusammen mit einem Corticosteroid, und in einem sechsten Aspekt die Verwendung eines Corticosteroids zur Herstellung einer pharmazeutischen Zusammensetzung zur Verwendung in einer Kombinationstherapie zusammen mit einem Cytokin-Antagonisten, einem weiteren Cytokin-Antagonisten und optional einem Wachstumsfaktor. Sowohl im Fall des fünften als auch des sechsten Aspekts der Erfindung ist der Cytokin-Antagonist der rekombinante Interleukin-Antagonist IL-1Ra-Protein, insbesondere Anakinra, der weitere Cytokin-Antagonist der natürlich vorkommende Interleukin-Antagonist IL-1Ra-Protein, und die pharmazeutische Zusammensetzung ist für eine lokale Verabreichung geeignet.

Weitere Ausführungsformen der Erfindung sind in der nachfolgenden ausführlichen Beschreibung und in den Patentansprüchen angegeben.

### Beschreibung der Erfindung

Die Erfindung basiert auf der überraschenden Erkenntnis, dass die Behandlung von Erkrankungen der Gelenke und der Wirbelsäule wie Arthrose, Arthritis, entzündlichen Formen der Arthrose und degenerative Wirbelsäulenerkrankung, sowie bei Autoimmunerkrankungen mittels Corticosteroiden durch die zusätzliche Verabreichung eines Cytokin-Antagonisten und optional eines Wachstumsfaktors deutlich verbessert werden kann. Insbesondere ergibt sich für die Behandlung mit dem rekombinanten IL-1Ra Anakinra erst durch die Kombination mit einem Corticosteroid eine Wirksamkeit, die um vieles höher ist als die alleinige Wirkung des Corticosteroids bzw. das Anakinra in der Kombination mit einem Corticosteroid überhaupt erst zu einem wirksamen Agens bei der Behandlung der genannten Erkrankungen macht. Bei dem natürlichen IL-1Ra Orthokin ist eine deutliche Verbesserung der Wirksamkeit gerade bei entzündlichen bzw. entzündlichen Verläufen der Arthrose oder Entzündungen der Wirbelgelenke oder der Nervenwurzel zu beobachten. Somit richtet sich die Erfindung auf die Kombinationstherapie solcher Erkrankungen mittels eines Corticosteroids zusammen mit einem Cytokin-Antagonisten und einem weiteren Cytokin-Antagonisten wie Anakinra bzw. Orthokin und optional eines Wachstumsfaktors. Die vorliegende Beschreibung offenbart ferner die Kombinationstherapie solcher Erkrankungen mittels eines Corticosteroids zusammen mit einem Cytokin-Antagonisten wie Anakinra oder Orthokin und optional eines Wachstumsfaktors.

Diese unterschiedlichen Wirkstoffe können dabei gleichzeitig - in derselben Formulierung oder in unterschiedlichen Formulierungen - oder auch sequentiell verabreicht werden. Die pharmazeutischen Zusammensetzungen, die nur einen der beiden unterschiedlichen Wirkstoffe enthalten, ebenso wie der Kit, können daher einerseits für eine simultane Verabreichung und andererseits für eine sequentielle Verabreichung des bzw. der Cytokin-Antagonisten und des Corticosteroids bestimmt sein. Eine gleichzeitige Verabreichung ist allerdings bevorzugt, insbesondere in nur einer Formulierung. So können zum Beispiel die beiden pharmazeutischen Zusammensetzungen des erfindungsgemäßen Kits vor einer Verabreichung an den Patienten in geeignetem Verhältnis gemischt werden und dann als eine Formulierung verabreicht werden. Bei einer sequentiellen Verabreichung des bzw. der Cytokin-Antagonisten und des Corticosteroids werden die verschiedenen Wirkstoffe vorzugsweise innerhalb eines Zeitraums von einer Woche, vorzugsweise innerhalb von 5 Tagen, 3 Tagen, einem Tag, oder innerhalb von 12 Stunden verabreicht.

Erfindungsgemäß können die Cytokin-Antagonisten auch mit einem Wachstumsfaktor kombiniert werden. In der erfindungsgemäßen pharmazeutischen Zusammensetzung bzw. dem erfindungsgemäßen Kit ist ein weiterer Cytokin-Antagonist enthalten.

Der gemäß der Beschreibung verwendete Cytokin-Antagonist kann ein jeglicher Stoff oder ein jegliches Stoffgemisch sein, das im Körper des Patienten mindestens eine, vorzugsweise im wesentlichen alle der biologischen Aktivitäten von einem oder mehreren Cytokinen verringert oder hemmt. Die antagonistische Wirkung kann dabei direkt durch den Antagonisten oder indirekt geschehen, zum Beispiel durch Aktivierung oder Hemmung weiterer Signalwege, die ihrerseits auf die biologische Aktivität des Cytokins einwirken. Vorzugsweise wird die biologische Aktivität des Cytokins durch Blockierung seiner Interaktion mit einem oder mehreren der Rezeptoren, an die es binden kann, gehemmt. Dies kann zum Beispiel durch kompetitive Bindung des Antagonisten an den/die entsprechenden Rezeptoren oder durch Bindung des Antagonisten an das Cytokin selbst erreicht werden. Vorzugsweise hemmt der Cytokin-Antagonist der Beschreibung die Wirkung des Cytokins IL-1.

Der Cytokin-Antagonist der Beschreibung kann beispielsweise ein Protein, ein Peptid, eine Nukleinsäure, ein Lipid oder eine organische Verbindung sein. Auch kann der Cytokin-Antagonist aus einem Gemisch von zwei oder mehr Cytokin-Antagonisten wie sie hierin beschrieben sind bestehen. Insbesondere kann der Cytokin-Antagonist ein natürlich vorkommendes Peptid oder Protein oder auch ein rekombinant hergestelltes Peptid oder Protein sein. Außerdem kann der Cytokin-Antagonist ein Antikörper oder ein Antigenbindendes Fragment eines Antikörpers sein oder enthalten, insbesondere ein Antikörper oder Antikörperfragment, das das betreffende Cytokin oder einen Cytokin-Rezeptor binden kann. Beispiele für geeignete Cytokin-Antagonisten sind Interleukin-Antagonisten, insbesondere IL-1-Antagonisten wie IL-1Ra, Tumornekrosefaktor (TNF)-Antagonisten, insbesondere ein TNF-α-Antagonist wie ein Anti-TNF-α-Antikörper, Interferon-Antagonisten, und Chemokin-Antagonisten. Besonders bevorzugt ist natürlich vorkommendes oder rekombinantes IL-1Ra-Protein, vorzugsweise humanes IL-1Ra. IL-1 Ra umfasst vorzugsweise oder besteht vorzugsweise aus der Aminosäuresequenz einer Isoform oder eines Homologs des humanen IL-1Ra gemäß SEQ ID NOs: 1, 2, 3, 4 oder 5, einer Isoform des equinen IL-1Ra gemäß SEQ ID NOs: 6 oder 7, oder einer Isoform des caninen IL-1Ra gemäß SEQ ID NO: 8. Vorzugsweise umfasst die pharmazeutische Zusammensetzung der Beschreibung oder der Kit der Beschreibung einen solchen Cytokin-Antagonist neben dem natürlich vorkommenden oder rekombinanten IL-1Ra-Protein.

Außerdem können gemäß der Beschreibung Fragmente oder Derivate von IL-1Ra als Cytokin-Antagonisten verwendet werden, solange diese die gewünschte Funktion, das heißt das Verringern oder Hemmen einer oder mehrerer biologischer Funktionen von IL-1, ausüben können. Fragmente von IL-1 Ra umfassen vorzugsweise mindestens 20, mehr bevorzugt mindestens 40, 60, 80 oder mindestens 100 Aminosäuren einer natürlichen IL-1 Ra-Sequenz. Vorzugsweise sind die Fragmente natürlicherweise vorkommende sekretierte Fragmente von IL-1Ra. In einer Ausführungsform umfasst das IL-1Ra die Aminosäuren 26 bis 177 des humanen IL-1 Ra, vorzugsweise Aminosäuren 26 bis 177 der Sequenz gemäß SEQ ID NO: 1. Derivate von IL-1Ra sind vorzugsweise homolog zu natürlichem IL-1Ra und haben bevorzugt eine Homologie oder Identität zu natürlichem IL-1 Ra von mindestens 60%, mehr bevorzugt mindestens 70%, 75%, 80%, 85%, 90%, 95% und am meisten bevorzugt mindestens 98% über einen Bereich von mindestens 20 zusammenhängenden Aminosäuren, bevorzugt mindestens 40, 60, 80 oder mindestens 100 zusammenhängenden Aminosäuren, am meisten bevorzugt über die gesamte Länge von IL-1Ra. Besonders bevorzugt sind das aus natürlichen biologischen Proben wie Blut isolierbare IL-1Ra, auch Orthokin genannt, sowie das IL-1Ra Fragment mit den Aminosäuren 26 bis 177 des humanen IL-1Ra, auch Anakinra genannt. Die Gewinnung von Orthokin ist unter anderem in den Patentanmeldungen WO 00/46249 A1 und WO 03/080122 A1 beschrieben. Anakinra sowie weitere IL-1-Antagonisten, die gemäß dieser Beschreibung verwendet werden können, sind unter anderem in der Patentanmeldung EP 0 343 684 A1 beschrieben.

Bei der Gewinnung von IL-1Ra aus natürlichen biologischen Proben wie Blut, wie z.B. bei Orthokin, enthält die erhaltene IL-1 Ra-Lösung vorzugsweise auch Wachstumsfaktoren, die auch für die überraschende Wirksamkeit der erfindungsgemäßen Wirkstoffkombination verantwortlich sein können. Daher können die Cytokin-Antagonisten erfindungsgemäß auch in Kombination mit einem oder mehreren Wachstumsfaktoren vorliegen oder durch einen oder mehrere Wachstumsfaktoren ersetzt werden. Der Wachstumsfaktor hat erfindungsgemäß vorzugsweise eine anabole Wirkung. Beispiele für geeignete Wachstumsfaktoren sind TGF-β, IGF, BMP, HGF und VEGF. Auch umfasst sind Analoge, Derivate und Fragmente dieser Wachstumsfaktoren, solange diese die gewünschte Wirkung, d.h. insbesondere ihre Wirkung als Wachstumsfaktor aufweisen.

Das erfindungsgemäß verwendete Corticosteroid kann ein jegliches natürlich vorkommendes sowie synthetisch hergestelltes Corticosteroid sein. Insbesondere kann es ein Glucocorticoid, ein Mineralocorticoid oder ein Androgen sein, wobei Glucocorticoide bevorzugt verwendet werden. Ebenso kann auch ein Gemisch aus zwei oder mehreren Corticosteroiden wie hierin beschrieben verwendet werden. Beispiele für Glucocorticoide sind Cortison, Hydrocortison, Prednison, Prednisolon, Cloprednol, Deflazacort, Fluocortin, Triamcinolon, Dexamethason, Methylprednisolon, Fluprednisolon, Clocortolon, Clobetason, Alclomethason, Flumethason, Fluopredniden, Fluorandrenolon, Betamethason, Beclomethason, Fluocortolon, Mometason, Fluticason, Halomethason, Fluocinolon, Diflorason, Desoximethason, Fluocinonid, Amcinonid, Halcinonid, Diflucortolon, Clobetasol und Paramethason. Beispiele für Mineralocorticoide sind Aldosteron, Desoxycorticosteron und Fludrocortison, und Beispiele für Androgene sind Dehydroepiandrosteron (DHEA) und Estrogene. Das Corticosteroid kann als freie Verbindung oder in Form eines Salzes, Esters oder Prodrugs eingesetzt werden. In bevorzugten Ausführungsformen ist das verwendete Corticosteroid Triamcinolon, Cortison, Hydrocortison, Prednisolon oder Prednison.

In bevorzugten Ausführungsformen sind die erfindungsgemäßen pharmazeutischen Zusammensetzungen bzw. der erfindungsgemäße Kit für eine Verwendung bei der Behandlung von Gelenkerkrankungen wie Arthrose, Arthritis, Gelenkentzündung und entzündlichem Knorpelverlust, Sehnenerkrankungen, degenerativen Wirbelsäulenerkrankungen und auch Autoimmunerkrankungen bestimmt. Die zu behandelnde Arthrose kann durch Überbelastung entstanden sein, angeborene oder traumatische Ursachen haben oder die Folge einer anderen Erkrankung wie einer Entzündung sein. Die zu behandelnde Arthrose ist vorzugsweise eine aktivierte Arthrose oder eine entzündliche Arthrose. Die erfindungsgemäßen pharmazeutischen Zusammensetzungen können für die Behandlung von Arthrosen und Arthritis an jedem beliebigen Gelenk eingesetzt werden, wie zum Beispiel Kniegelenk, Hüftgelenk, Sprunggelenk, Schultergelenk, Wirbelgelenke, Fingergelenke, Ellenbogengelenk, Zehengelenke, Kiefergelenk und Handgelenk. Die zu behandelnde Arthritis kann eine infektionsbedingte Arthritis wie bakterielle Arthritis oder eine nicht-infektionsbedingte Arthritis wie rheumatoide Arthritis, psoriatische Arthritis oder Gicht-Arthritis sein. Alternativ können die erfindungsgemäße pharmazeutische Zusammensetzung und/oder der erfindungsgemäße Kit auch zur Verwendung bei der Behandlung einer von einer oder mehreren der genannten Krankheiten (etwa rheumatoider Arthritis) verschiedenen Krankheit bestimmt sein. Die zu behandelnde degenerative Wirbelsäulenerkrankung kann beispielsweise ein Bandscheibenvorfall sein. Autoimmunerkrankungen umfassen unter anderem Autoimmunerkrankungen der Gelenke wie zum Beispiel Morbus Bechterew, rheumatoide Arthritis und systemischer Lupus erythematodes, sowie andere Autoimmunerkrankungen wie insbesondere Neurodermitis und Alopecia areata (kreisrunder Haarausfall).

Die erfindungsgemäßen pharmazeutischen Zusammensetzungen bzw. der erfindungsgemäße Kit sind vorzugsweise zur lokalen Verabreichung bestimmt. Daher sind sie in bevorzugten Ausführungsformen zur Injektion, insbesondere zur Injektion in die zu behandelnde Körperregion, insbesondere in das betroffene Gelenk, an die betroffene Nervenwurzel oder in die betroffene Bandscheibe, oder in die lokale Umgebung hiervon bestimmt. Die pharmazeutische Zusammensetzung ist somit insbesondere zur intraartikulären und/oder periradikulären Injektion bestimmt. Alternativ können die erfindungsgemäßen pharmazeutischen Zusammensetzungen für eine topische Verabreichung formuliert sein, insbesondere als Creme oder Gel, oder für eine systemische Verabreichung, insbesondere eine orale Verabreichung in Form von Tabletten, Kapseln oder Pastillen. Die Verabreichungsform hängt unter anderem von der zu behandelnden Krankheit ab. Bei einer lokalen Arthrose oder einer degenerativen Wirbelsäulenerkrankung ist daher eine lokale Verabreichung der erfindungsgemäßen pharmazeutischen Zusammensetzungen bevorzugt. In bevorzugten Ausführungsformen sind die erfindungsgemäßen pharmazeutischen Zusammensetzungen bzw. der erfindungsgemäße Kit ausschließlich für eine von einer systemischen Verabreichung verschiedene Verabreichung bestimmt oder geeignet.

Die erfindungsgemäßen pharmazeutischen Zusammensetzungen sind für die verschiedenen Verabreichungswege jeweils geeignet in dem Fachmann bekannter Weise formuliert. So liegt eine für eine Injektion geeignete pharmazeutische Zusammensetzung vorzugsweise als Lösung oder Dispersion oder auch in trockener Form z.B. als Pulver oder Lyophilisat vor, welches vor der Injektion in einem geeigneten Lösungsmittel wie Wasser gelöst werden muss. Die erfindungsgemäßen pharmazeutischen Zusammensetzungen enthalten den Cytokin-Antagonisten und/oder das Corticosteroid in therapeutisch wirksamen Mengen. Der Cytokin-Antagonist ist daher vorzugsweise in einer Konzentration von 0,5 bis 150 mg/Dosis in den Cytokin-Antagonist-enthaltenden pharmazeutischen Zusammensetzungen vorhanden, kann aber auch in erheblich geringerer Konzentration wie etwa 1 ng/Dosis oder mehr, zum Beispiel zwischen 1 und 1000 ng/Dosis vorliegen. Diese geringen Dosiskonzentrationen können insbesondere bei einer Kombination mit Wachstumsfaktoren und/oder bei natürlichen IL-1Ra-Präparaten wie beispielsweise bei Zusammensetzungen mit Orthokin verwendet werden. Die höheren Dosiskonzentrationen sind beispielsweise bei rekombinant hergestellten Cytokin-Antagonisten wie Anakinra bevorzugt. Das Corticosteroid liegt vorzugsweise in einer Konzentration von 1 bis 80 mg/Dosis, mehr bevorzugt von 5 bis 40 mg/Dosis in den Corticosteroid-enthaltenden pharmazeutischen Zusammensetzungen vor. Außerdem können die erfindungsgemäßen pharmazeutischen Zusammensetzungen ferner einen oder mehrere Trägerstoffe und/oder ein oder mehrere Exzipienten enthalten.

Die pharmazeutischen Zusammensetzungen der Erfindung können auch für eine Behandlung von Patienten bestimmt sein, die bereits einer anderen Behandlung der relevanten Krankheit, d.h. zum Beispiel einer Arthrose, Arthritis, und/oder degenerativen Wirbelsäulenerkrankung, unterzogen wurden, insbesondere wenn diese andere Behandlung nicht erfolgreich war oder die Krankheitssymptome trotz anfänglich erfolgreicher Behandlung zumindest teilweise zurückgekehrt sind. In bevorzugten Ausführungsformen ist diese andere Behandlung eine Therapie mit einem Cytokin-Antagonisten wie zu Beispiel Anakinra oder Orthokin, aber ohne ein Corticosteroid, oder eine Therapie mit einem Corticosteroid, insbesondere einem Glucocorticoid wie vorstehend beschrieben, aber ohne einen Cytokin-Antagonisten.

Patienten im Sinne der Erfindung können Menschen sowie Tiere sein, die unter einer der hierin beschriebenen Krankheiten leiden. Somit können die erfindungsgemäßen pharmazeutischen Zusammensetzungen geeignet sein für die Behandlung eines Menschen und/oder eines Tieres wie zum Beispiel eines Hundes, einer Katze, eines Pferdes, eines Rinds, eines Schweins, einer Ziege, eines Kamels oder dergleichen.

In einer weiteren Variante sind die pharmazeutischen Zusammensetzungen der Beschreibung für eine Verwendung in einer Kombinationstherapie zusammen mit Exosomen bestimmt. Exosome sind kleine, von einer Lipidmembran umhüllte Vesikel, die im extrazellulären Raum zum Beispiel des menschlichen Körpers vorkommen. Sie werden von Zellen durch Abspaltung von der zellulären Plasmamembran gebildet und sekretiert. Normalerweise enthalten diese Exosomen auch Proteine, die sie von ihrer Ursprungszelle übernommen haben.

Die Exosomen können direkt in der pharmazeutischen Zusammensetzung der Beschreibung enthalten sein oder werden in einer separaten Zusammensetzung simultan oder sequentiell verabreicht. Vorzugsweise werden die Exosomen aus einer Blutprobe gewonnen, wobei die Exosomen bezüglich des zu behandelnden Patienten vorzugsweise autolog oder allogen sind. Verfahren zur Gewinnung und Verabreichung von Exosomen sind zum Beispiel in der Patentanmeldung WO 2006/007529 A2 beschrieben.

Folglich sieht eine gemäß der Beschreibung bevorzugte Variante vor, dass die pharmazeutischen Zusammensetzungen der Beschreibung für eine Behandlung bestimmt sind, bei der zuerst Exosomen aus einer Blutprobe eines Patienten gewonnen werden und diese anschließend zusammen mit einem Cytokin-Antagonisten und einem Corticosteroid diesem Patienten wieder verabreicht werden.

Für den Fall, dass die Kombinationstherapie der Beschreibung Exosomen involviert, die aus einer Blutprobe eines Patienten gewonnen werden, ist es in manchen Fällen bevorzugt, einen Zentrifugationsschritt mit mindestens 100 000 g durchzuführen, um die Exosomen aufzukonzentrieren, da solch hohe relative Zentrifugalbeschleunigungen besonders geeignet sind, um Exosomen zu konzentrieren. Vorzugsweise wird ein solcher Zentrifugationsschritt bei der Behandlung von Krankheiten durchgeführt, bei denen eine erhöhte Konzentration von Exosomen sinnvoll ist, bevorzugt bei der Behandlung von rheumatoider Arthritis. Besonders bevorzugt wird ein solcher Zentrifugationsschritt ganz allgemein durchgeführt für den Fall, dass die Kombinationstherapie Exosomen involviert, die aus einer Blutprobe eines Patienten gewonnen werden. Der Zentrifugationsschritt mit mindestens 100 000 g wird vorzugsweise mindestens 30 min, insbesondere mindestens 60 min durchgeführt, da das Aufkonzentrieren dann besonders effektiv ist.

### Beispiele

Im Folgenden sind verschiedene Fallstudien von Patienten mit fortgeschrittener Arthrose beschrieben. Diese wurden mit einer Kombinationstherapie mit einem Cytokin-Antagonisten (z.B. rekombinantes IL-1Ra rekombinant oder aus autologen Blutproben gewonnenes IL-1 Ra) und einem Corticosteroid behandelt.

### Abkürzungen:

- re: rechts
- li: links
- bds: beidseitig
- IRO: Innenrotation
- ARO: Außenrotation
- VAS: visuelle Analogskala für Schmerzempfindung (0 bis 10)
- WOMAC: Patientenfragebogen zu Arthrose
- CRP: c-reaktives Protein, ein im Blut nachweisbarer Entzündungsmarker
- OSG: Oberschenkelgelenk

### 1. Lokale Verabreichung von Anakinra und Cortison

### Fall I: A. , 69 Jahre

*Diagnose:* Coxarthrose re, Grad III-IV im Röntgenbild; Hüftschmerzen mit Hinken seit ca. 3 Jahren, Patientin will noch keinen Hüftersatz; klinisches Schonhinken, IRO/ARO re Hüfte 5/0/5, nebenbefundlich Borreliose bekannt;
*Therapie:* Es wurden 5x wöchentliche Injektionen von 1 mg Anakinra mit 10 mg Triamcinolon verabreicht.
*Ergebnis:* Beim Therapieende (nach der 5. Sitzung) war das Schonhinken verschwunden. IRO/ARO rechts jetzt 10/0/15; VAS von 8 auf 3 verbessert; 70% Schmerzreduktion (persönliche Einschätzung des Patienten nach momentanem Schmerzgrad (hier 30%) im Vergleich zum Schmerz vor der Behandlung (100%), die Schmerzreduktion ist die Differenz zwischen momentanem Schmerz und Schmerz vor der Behandlung (100% - 30% = 70%)). Nach 3-monatiger Kontrolle unveränderte Besserung im Vergleich zum Status bei Therapieende.

### Fall II: R., 54 Jahre, weiblich

*Diagnose:* klinisch und radiologisch Rhizarthrose re mittelgradig mit starken Schmerzen (VAS 6) mit Funktionsbehinderung beim Greifen von Gegenständen. Cortisoninjektionen in der Vergangenheit ohne Erfolg.

*Therapie:* Es wurde eine 5-malige Injektionsbehandlung für das rechte Daumensattelgelenk aus 0,5 mg Anakinra in Kombination mit 1 mg Triamcinolon (bei der 1., 3. und 5. Sitzung) vorgenommen.

*Ergebnis:* Bei Therapieende Schmerzfreiheit, 100% Besserung; VAS jetzt 0; Funktion der rechten Hand normal; bei der Kontrolle 3 Monate nach Therapieende weiter unverändert sehr gutes Ergebnis.

### Fall III: W., 49 Jahre, männlich

*Diagnose:* Klinisch und radiologisch mittelgradige Kniearthrose bds. Grad II-III seit vielen Jahren. Hyaloronsäureinjektionen und Cortisoninjektionen in die Knie in der Vergangenheit ohne Erfolg

*Therapie:* Es wurde eine 6-malige Anakinrabehandlung mit Anwendung von 10 mg Triamcinolon bei der 1., 3. und 5 Sitzung vorgenommen.

*Ergebnis:* Bei Therapieende 100% Schmerzbesserung re Knie, linkes Knie 70% Schmerzbesserung

### Fall IV: T., 45 Jahre, männlich, Musiker

*Diagnose:* Radiologisch und klinisch Inpingement li Schulter seit ca. 2 Jahren; vormalige Cortisoninjektionen ohne Erfolg; Abduktion um ca. 15 Grad eingeschränkt

*Therapie:* Es wurde eine Injektion von 50 mg Anakinra mit 10 mg Triam sowie und eine Kontrolle vorgenommen. Die Injektion von Anakinra und 10 mg Triam wurde in einer Spritze aufgemischt und steril aufgezogen.

*Ergebnis:* Bei der nachfolgenden Kontrolle eine Woche nach der Behandlung komplette Schmerzfreiheit (100%), Funktion normal. Wegen des guten Therapieerfolges wurden keine weiteren Behandlungen geplant. Folgekontrollen waren unauffällig.

### Fall V: K., 45 Jahre, männlich

*Diagnose:* Klinisch und radiologisch mediale Kniearthrose re Grad IV und retropatellar seit vielen Jahren; auswärtig wurde der Versuch einer Umstellungsosteotomie oder ein Knieersatz rechts empfohlen. Patient wünschte allerdings einen konservativen Therapieversuch. In der Vergangenheit waren Injektionen von Hyaloronsäure und Cortison (Triamcinolon) ohne klinischen Erfolg.

*Therapie:* Es wurde eine 10-malige Anakinrabehandlung (100 mg je Sitzung) mit paralleler Verabreichung von 10 mg Triamcinolon (Behandlungsgesamtdosis 50 mg) 2-mal wöchentlich durchgeführt.

*Ergebnis:* Zum Therapieende 65% Schmerzreduktion nach 3 Monaten.

### Fall VI: M., 50 Jahre, männlich

*Diagnose:* Klinisch und radiologisch (MRT) Innemeniskusschaden re Knie III mit deutlicher Funktionsverschlechterung und Schmerz medial rechtes Knie. OP wurde empfohlen, Patient möchte gerne nicht operative Alternative.

*Therapie:* Es wurde eine einmalige Injektion von 10 mg Triamcinolon und 1 mg Anakinra vorgenommen.

*Ergebnis:* Ein Monat nach Injektion 80% Schmerzbesserung, Patient will keine OP mehr, sondern erneute Injektion, da diese sehr gut geholfen habe. Bei der Nachkontrolle 6 Monate nach der Therapie weiterhin kein OP Wunsch, Patient schmerzfrei.

### Fall VII: G., 42 Jahre, männlich

*Diagnose:* Klinisch und radiologisch lumable Fazettenarthrose seit vielen Jahren, nebenbefundlich Divertikel. Stationäre Behandlung mit Cortisoninjektionen in die lumbalen Fazetten ohne Erfolg.

*Therapie:* 6 Therapiesitzungen 2x wöchentlich mit Injektionen von 6 mg Anakinra in die lumbalen Fazetten wurden vorgenommen. Bei der ersten Injektion wurden zusätzlich 3 mg Triamcinolon, bei den Folgebehandlungen 2-6 wurden ausschließlich 6 mg Anakinra verabreicht.

*Ergebnis:* 60% Schmerzbesserung, VAS gebessert von 7 vor Behandlung auf 3 nach Behandlung. Kontrollen unverändert nach 5 Monaten.

**Tabelle 1: Therapie mit Anakinra und Cortison. Statistische Auswertung einer Fallserie**

| **Schmerzlevel vorher** | **Schmerzlevel nachher** |
|---|---|
| 100 | 30 |
| 100 | 0 |
| 100 | 30 |
| 100 | 0 |
| 100 | 35 |
| 100 | 0 |
| 100 | 40 |
| 100 | 60 |
| 100 | 40 |
| 100 | 50 |
| 100 | 80 |
| 100 | 50 |

Patientenzahl: N = 12
durchschnittliche Schmerzreduktion: 71,5% nach ca. 3 Monaten (vor Therapie 100% Schmerz, Therapieende 28,5% Schmerz)
Standardabweichung: SD = 22
p < 0,001

### Therapie mit Orthokin und Cortison bei Arthrose:

Patientenzahl: N = 129
durchschnittlicher Nachuntersuchungszeitraum: 3 Monate
durchschnittliche Schmerzreduktion: 71% (d.h. Reduktion von 100% Schmerz vor Behandlung auf 29% nach Behandlung)
   auffallend schneller Wirkungseintritt

### 2. Lokale Verabreichung von Anakinra und Cortison und Exosomen

### Fall VIII: T., 56 Jahre, weiblich

*Diagnose:* Klinisch radiologisch besteht mediale und retropatellare Gonarthrose li, Grad IV. Auswärtig wurde bereits ein totaler Knieersatz links geplant;
*Therapie:* 3 Injektion von Exosomen kombiniert mit Anakinra und 10 mg Triamcinolon in das linke Knie (2x wöchentlich) unter Vermeidung einer Knieoperation.
*Ergebnis:* Zum Zeitpunkt der 3. Injektion 100% Schmerzbesserung, deutliche Funktionsverbesserung. Operationstermin wurde abgesagt, Patientin war auch 5 Monate nach Therapieende noch schmerzfrei.

### 3. Lokale Verabreichung von Anakinra und Cortison und Orthokin

### Fall IX: L., 57 Jahre, männlich

*Diagnose:* Starke Schulterschmerzen li seit 6 Monaten (VAS 8); seitdem deutlich gestörte Nachtruhe. Patient konnte in den letzten 6 Monaten kaum schlafen, deswegen auch Störung des allgemeinen Wohlbefindens. Zahlreiche Injektionen mit Cortison in die linke Schulter waren ohne Erfolg. Ein Operationstermin für die linke Schulter war vereinbart. Hier sollte ein Versuch unternommen werden, die Operation zu vermeiden. Radiologisch und klinisch Zeichen der partiellen Rotatorenmanschettenruptur und der subakromialen Enge mit kompletter Schultersteife links; Missempfindungen linker Arm mit Schwäche der Kraft von Hand und Unterarm links, Grad 4.

*Therapie:* Die Injektionen wurden in die linke Schulter dorsal und lateral appliziert. 2 ml Orthokin wurden mit 10 mg Anakinra und 10 mg Triamcinolon über eine Spritze in die linke Schulter appliziert. Die Therapie wurde an 4 aufeinander folgenden Tagen durchgeführt.

*Ergebnis:* Bereits am 2. Behandlungstag gab der Patient eine extreme Schmerzbesserung mit einer Schmerzreduktion um 90% an. VAS war von 8 auf 1 gefallen, die Schulter war frei und normal beweglich. Der Patient konnte erstmalig seit 6 Monaten durchgeschlafen. Der Patient war dadurch in seinem allgemeinen Wohlbefinden deutlich gebessert. Fortsetzung der Therapie bis Behandlungstag 4. Weiterhin zeigte sich eine unverändert deutliche Besserung wie an Behandlungstag 2, bei der Nachkontrolle 6 Monate nach Behandlung gab es einen unverändert positiven Befund. Die Operation wurde abgesagt, Beweglichkeit war frei, der Patient kann wieder problemlos Koffer und Bücher über Schulterhöhe hinaus heben.

### Fall X: F., 45 Jahre, weiblich

*Diagnose:* Komplette Schultersteife rechts seit ca. 8 Monaten. Alle bisherigen Therapien waren ohne Erfolg, eine OP war geplant. Patientin wollte Versuch der erneuten konservativen Behandlung. Der Nachtschlaf war seit einigen Wochen nicht mehr möglich. Links beginnende Schulterschmerzen, Hauptbefund aber rechte Schulter, dort VAS 9 mit schweren akuten Attacken bis auf 10, dadurch insgesamt reduzierter Allgemeinzustand. *Therapie:* Behandlung der rechten Schulter aus einer Kombination von 2 ml Orthokin getrennt appliziert zusammen mit einer anderen Spritze aus einer Kombination von 150 mg Anakinra und 5 mg Triamcinolon an 6 aufeinander folgenden Tagen.

*Ergebnis:* Ab dem 5. Tag 85% Schmerzbesserung. Nächtliches Durchschlafen war seit 2. Behandlung möglich, dadurch erheblich gebesserter Allgemeinzustand. VAS bei Behandlungsende bei 1. Nachkontrolle 8 Monate nach Behandlung zeigte weiter ein sehr gutes unverändertes Ergebnis; OP wurde abgesagt.

### 4. Exosomen inkubiert mit IL-1Ra und Triamcinolon/Prednisolon

### Fall X: S. 25 Jahre, männlich

*Diagnose:* Schwere juvenile rheumatoide Arthritis seit ca. 15 Jahren.
Behandlung mit 25 mg Enbrel 2 x pro Woche, 10 mg Methotrexat, 5 mg Decortin und Naproxen 2x1 pro Tag. Massive Synovitis und Schmerzen beide OSG und beide Schultern. Abduktion beider Schultern vor Behandlung 60 Grad. Labor CRP-Wert: 5,35 (bis 0,5 mg ist normal); Leukozytose.

*Therapie:* Es wurde Blut abgenommen zur Herstellung von Exosomen in einer 6 ml Spritze (Orthokinspritze). Danach 24 h Inkubation bei 37 Grad, wobei bei Füllung der Spritze mit Blut vorher 1 mg Anankinra (IL-1Ra) und 2 mg Prednisolon in die Spritze appliziert wurden. Nach verschiedenen Schritten der Zentrifugation (bis 100 000 g) wurde das Gemisch dann der Patientin in die OSGe und die Schultern appliziert.

*Ergebnis:* Nach 3 Tagen beginnende deutliche Abschwellung der Gelenke. Klinische und laborchemische Kontrolle nach 9 Tagen: 80% Schmerzbesserung, OSG normal, keine Schwellung. CRP-Wert jetzt auf 1,93. Verbesserung auch in anderen betroffenen Gelenken, die nicht lokal injiziert wurden. Allgemeine Lebensqualität erheblich verbessert. Bei der Kontrolle nach 3 Monaten zeigt sich eine unverändert stabile Situation. VAS vor Behandlung 9, seit der ersten Woche nach Injektion VAS 3. Patient sehr zufrieden, kann Arbeit wieder fortsetzen.

## Patentansprüche

1. Pharmazeutische Zusammensetzung enthaltend ein Corticosteroid zusammen mit einem Cytokin-Antagonisten, einem weiteren Cytokin-Antagonisten und optional einem Wachstumsfaktor, wobei der Cytokin-Antagonist der rekombinante Interleukin-Antagonist IL-1Ra-Protein ist, insbesondere Anakinra, der weitere Cytokin-Antagonist der natürlich vorkommende Interleukin-Antagonist IL-1Ra-Protein ist, insbesondere Orthokin, und wobei die pharmazeutische Zusammensetzung für eine lokale Verabreichung geeignet ist.

2. Pharmazeutische Zusammensetzung enthaltend einen Cytokin-Antagonisten, einen weiteren Cytokin-Antagonisten und optional einen Wachstumsfaktor zur Verwendung in einer Kombinationstherapie zusammen mit einem Corticosteroid, wobei der Cytokin-Antagonist der rekombinante Interleukin-Antagonist IL-1Ra-Protein ist, insbesondere Anakinra, der weitere Cytokin-Antagonist der natürlich vorkommende Interleukin-Antagonist IL-1Ra-Protein ist, insbesondere Orthokin, und wobei die pharmazeutische Zusammensetzung für eine lokale Verabreichung geeignet ist.

3. Pharmazeutische Zusammensetzung enthaltend ein Corticosteroid zur Verwendung in einer Kombinationstherapie zusammen mit einem Cytokin-Antagonisten, einem weiteren Cytokin-Antagonisten und optional einem Wachstumsfaktor, wobei der Cytokin-Antagonist der rekombinante Interleukin-Antagonist IL-1Ra-Protein ist, insbesondere Anakinra, der weitere Cytokin-Antagonist der natürlich vorkommende Interleukin-Antagonist IL-1Ra-Protein ist, insbesondere Orthokin, und wobei die pharmazeutische Zusammensetzung für eine lokale Verabreichung geeignet ist.

4. Kit enthaltend eine pharmazeutische Zusammensetzung enthaltend einen Cytokin-Antagonisten und optional einen Wachstumsfaktor, eine pharmazeutische Zusammensetzung enthaltend ein Corticosteroid, und außerdem einen weiteren Cytokin-Antagonisten, wobei der Cytokin-Antagonist der rekombinante Interleukin-Antagonist IL-1Ra-Protein ist, insbesondere Anakinra, der weitere Cytokin-Antagonist der natürlich vorkommende Interleukin-Antagonist IL-1Ra-Protein ist, insbesondere Orthokin, und wobei die pharmazeutischen Zusammensetzungen für eine lokale Verabreichung geeignet sind.

5. Pharmazeutische Zusammensetzung gemäß Anspruch 2 oder 3 oder Kit gemäß Anspruch 4, wobei die pharmazeutische(n) Zusammensetzung(en) für eine simultane oder eine sequenzielle Verabreichung des Cytokin-Antagonisten bzw. des Wachstumsfaktors und des Corticosteroids bestimmt ist (sind).

6. Pharmazeutische Zusammensetzung oder Kit gemäß einem der Ansprüche 1, 2 und 4 bis 5, wobei der Cytokin-Antagonist in einer Konzentration von 0,5 bis 150 mg/Dosis in der Cytokin-Antagonist-enthaltenden pharmazeutischen Zusammensetzung vorliegt.

7. Pharmazeutische Zusammensetzung oder Kit gemäß einem der Ansprüche 1 bis 6, wobei das Corticosteroid
(a) ein Glucocorticoid wie beispielsweise Cortison, Hydrocortison, Prednison, Prednisolon, Cloprednol, Deflazacort, Fluocortin, Triamcinolon, Dexamethason, Methylprednisolon, Fluprednisolon, Clocortolon, Clobetason, Alclomethason, Flumethason, Fluopredniden, Fluorandrenolon, Betamethason, Beclomethason, Fluocortolon, Mometason, Fluticason, Halomethason, Fluocinolon, Diflorason, Desoximethason, Fluocinonid, Amcinonid, Halcinonid, Diflucortolon, Clobetasol, Paramethason;
(b) ein Mineralocorticoid wie beispielsweise Aldosteron, Desoxycorticosteron und Fludrocortison; oder
(c) ein Androgen wie beispielsweise Dehydroepiandrosteron (DHEA) und Estrogene;
oder ein Salz, Ester oder Prodrug davon ist.

8. Pharmazeutische Zusammensetzung oder Kit gemäß einem der Ansprüche 1 und 3 bis 7, wobei das Corticosteroid in einer Konzentration von 1 bis 80 mg/Dosis in der Corticosteroid-enthaltenden pharmazeutischen Zusammensetzung vorliegt.

9. Pharmazeutische Zusammensetzung oder Kit gemäß einem der Ansprüche 1 bis 8, wobei der Wachstumsfaktor ausgewählt ist aus der Gruppe bestehend aus TGF-β, IGF, BMP, HGF und VEGF.

10. Pharmazeutische Zusammensetzung oder Kit gemäß einem der Ansprüche 1 bis 9 zur Verwendung bei der Behandlung von Gelenkerkrankungen wie Arthrose und entzündlichem Knorpelverlust, Sehnenerkrankungen und/oder degenerativen Wirbelsäulenerkrankungen.

11. Pharmazeutische Zusammensetzung oder Kit zur Verwendung gemäß Anspruch 10, wobei die Arthrose eine aktivierte Arthrose oder eine entzündliche Arthrose ist.

12. Pharmazeutische Zusammensetzung oder Kit zur Verwendung gemäß Anspruch 10, wobei die degenerative Wirbelsäulenerkrankung ein Bandscheibenvorfall ist.

13. Pharmazeutische Zusammensetzung oder Kit gemäß einem der Ansprüche 1 bis 9 zur Verwendung bei der Behandlung von Neurodermitis oder alopecia areata.

14. Pharmazeutische Zusammensetzung oder Kit gemäß einem der Ansprüche 1 bis 9, oder pharmazeutische Zusammensetzung oder Kit zur Verwendung gemäß einem der Ansprüche 10 bis 13, wobei die lokale Verabreichung ausgewählt ist aus der Gruppe bestehend aus Injektion in die betroffene Körperregion, insbesondere in das betroffene Gelenk, an die betroffene Nervenwurzel oder in die betroffene Bandscheibe, oder in die lokale Umgebung hiervon; intraartikuläre Injektion; und topische Verabreichung.

15. Pharmazeutische Zusammensetzung oder Kit gemäß einem der Ansprüche 1 bis 9 und 14, oder pharmazeutische Zusammensetzung oder Kit zur Verwendung gemäß einem der Ansprüche 10 bis 14, wobei die pharmazeutische(n) Zusammensetzung(en) ferner einen Träger und/oder ein Exzipienten enthält (enthalten).

16. Verwendung eines Cytokin-Antagonisten, einen weiteren Cytokin-Antagonisten und optional eines Wachstumsfaktors zur Herstellung einer pharmazeutischen Zusammensetzung zur Verwendung in einer Kombinationstherapie zusammen mit einem Corticosteroid, wobei der Cytokin-Antagonist der rekombinante Interleukin-Antagonist IL-1Ra-Protein ist, insbesondere Anakinra, der weitere Cytokin-Antagonist der natürlich vorkommende Interleukin-Antagonist IL-1 Ra-Protein ist, insbesondere Orthokin, und wobei die pharmazeutische Zusammensetzung für eine lokale Verabreichung geeignet ist.

17. Verwendung eines Corticosteroids zur Herstellung einer pharmazeutischen Zusammensetzung zur Verwendung in einer Kombinationstherapie zusammen mit einem Cytokin-Antagonisten, einem weiteren Cytokin-Antagonisten und optional einem Wachstumsfaktor, wobei der Cytokin-Antagonist der rekombinante Interleukin-Antagonist IL-1 Ra-Protein ist, insbesondere Anakinra, der weitere Cytokin-Antagonist der natürlich vorkommende Interleukin-Antagonist IL-1Ra-Protein ist, insbesondere Orthokin, und wobei die pharmazeutische Zusammensetzung für eine lokale Verabreichung geeignet ist.

## Claims

1. Pharmaceutical composition containing a corticosteroid together with a cytokine antagonist, a further cytokine antagonist and optionally a growth factor, wherein the cytokine antagonist is the recombinant interleukin antagonist IL-1RA protein, especially anakinra, the further cytokine antagonist is the naturally occurring interleukin antagonist IL-1RA protein, especially orthokine, and wherein the pharmaceutical composition is suitable for a local application.

2. Pharmaceutical composition containing a cytokine antagonist, a further cytokine antagonist and optionally a growth factor for use in a combination therapy together with a corticosteroid, wherein the cytokine antagonist is the recombinant interleukin antagonist IL-1RA protein, especially anakinra, the further cytokine antagonist is the naturally occurring interleukin antagonist IL-1RA protein, especially orthokine, and wherein the pharmaceutical composition is suitable for a local application.

3. Pharmaceutical composition containing a corticosteroid for use in a combination therapy together with a cytokine antagonist, a further cytokine antagonist and optionally a growth factor, wherein the cytokine antagonist is the recombinant interleukin antagonist IL-1RA protein, especially anakinra, the further cytokine antagonist is the naturally occurring interleukin antagonist IL-1RA protein, especially orthokine, and wherein the pharmaceutical composition is suitable for a local application.

4. Kit containing a pharmaceutical composition containing a cytokine antagonist and optionally a growth factor, a pharmaceutical composition containing a corticosteroid, and furthermore, a further cytokine antagonist, wherein the cytokine antagonist is the recombinant interleukin antagonist IL-1RA protein, especially anakinra, the further cytokine antagonist is the naturally occurring interleukin antagonist IL-1RA protein, especially orthokine, and wherein the pharmaceutical compositions are suitable for a local application.

5. Pharmaceutical composition according to claim 2 or 3 or kit according to claim 4, wherein the pharmaceutical composition(s) is (are) determined for a simultaneous or sequential application of the cytokine antagonist or respectively of the growth factor and of the corticosteroid.

6. Pharmaceutical composition or kit according to one of claims 1, 2 and 4 to 5, wherein the cytokine antagonist is present in a concentration from 0.5 to 150 mg/dose in the cytokine-antagonist-containing pharmaceutical composition.

7. Pharmaceutical composition or kit according to one of claims 1 to 6, wherein the corticosteroid is
(a) a glucocorticoid, such as a cortisone, hydrocortisone, prednisone, prednisolone, cloprednol, deflazacort, fluocortin, triamcinolone, dexa-methasone, methylprednisolone, fluprednisolone, clocortolone, clobetasone, alclomethasone, flumethasone, fluprednidene, flurandrenolone, beta-methasone, beclomethasone, fluocortolone, mometasone, fluticasone, halo-methasone, fluocinolone, diflorasone, desoximethasone, fluocinonide, amcinonide, halcinonide, diflucortolone, clobetasol, paramethasone;
(b) a mineralocorticoid, such as aldosterone, desoxycorticosterone and fludrocortisone; or
(c) an androgen, such as dehydroepiandrosterone (DHEA) and estrogens; or a salt, ester or prodrug thereof.

8. Pharmaceutical composition or kit according to one of claims 1 and 3 to 7, wherein the corticosteroid is present in a concentration from 1 to 80 mg/dose in the corticosteroid-containing pharmaceutical composition.

9. Pharmaceutical composition or kit according to one of claims 1 to 8, wherein the growth factor is selected from the group consisting of TGF-β, IGF, BMP, HGF and VEGF.

10. Pharmaceutical composition or kit according to one of claims 1 to 9 for use in the treatment of joint diseases, such as arthrosis and inflammatory cartilage loss, tendon diseases and/or degenerative vertebral diseases.

11. Pharmaceutical composition or kit for use according to claim 10, wherein the arthrosis is an activated arthrosis or an inflammatory arthrosis.

12. Pharmaceutical composition or kit for use according to claim 10, wherein the degenerative vertebral disease is a vertebral disc prolapse.

13. Pharmaceutical composition or kit according to one of claims 1 to 9 for use in the treatment of neurodermatitis or alopecia areata.

14. Pharmaceutical composition or kit according to one of claims 1 to 9, or pharmaceutical composition or kit for use according to one of claims 10 to 13, wherein the local application is selected from the group consisting of injection into the affected body region, especially into the affected joint, into the affected nerve root or into the affected vertebral disc, or into the local environment thereof; intra-articular injection; and topical application.

15. Pharmaceutical composition or kit according to one of claims 1 to 9 and 14, or pharmaceutical composition or kit for use according to one of claims 10 to 14, wherein the pharmaceutical composition(s) further contain(s) a carrier and/or an excipient.

16. Use of a cytokine antagonist, a further cytokine antagonist and optionally a growth factor for the manufacture of a pharmaceutical composition for use in a combination therapy together with a corticosteroid, wherein the cytokine antagonist is the recombinant interleukin antagonist IL-1RA protein, especially anakinra, the further cytokine antagonist is the naturally occurring interleukin antagonist IL-1RA protein, especially orthokine, and wherein the pharmaceutical composition is suitable for a local application.

17. Use of a corticosteroid for the manufacture of a pharmaceutical composition for use in a combination therapy together with a cytokine antagonist, a further cytokine antagonist and optionally a growth factor, wherein the cytokine antagonist is the recombinant interleukin antagonist IL-1RA protein, especially anakinra, the further cytokine antagonist is the naturally occurring interleukin antagonist IL-1RA protein, especially orthokine, and wherein the pharmaceutical composition is suitable for a local application.

## Revendications

1. Composition pharmaceutique contenant un corticoïde conjointement avec un antagoniste de cytokine, un autre antagoniste de cytokine et facultativement un facteur de croissance, dans laquelle l'antagoniste de cytokine est l'antagoniste d'interleukine protéine IL-1Ra recombinante, en particulier l'anakinra, l'autre antagoniste de cytokine est l'antagoniste d'interleukine protéine IL-1Ra naturelle, en particulier l'orthokine, et la composition pharmaceutique convenant pour une administration locale.

2. Composition pharmaceutique contenant un antagoniste de cytokine, un autre antagoniste de cytokine et facultativement un facteur de croissance pour l'utilisation dans un traitement combiné conjointement avec un corticoïde, dans laquelle l'antagoniste de cytokine est l'antagoniste d'interleukine protéine IL-1Ra recombinante, en particulier l'anakinra, l'autre antagoniste de cytokine étant l'antagoniste d'interleukine protéine IL-1Ra naturelle, en particulier l'orthokine, et la composition pharmaceutique convenant pour une administration locale.

3. Composition pharmaceutique contenant un corticoïde pour l'utilisation dans un traitement combiné conjointement avec un antagoniste de cytokine, un autre antagoniste de cytokine et facultativement un facteur de croissance, dans laquelle l'antagoniste de cytokine est l'antagoniste d'interleukine protéine IL-1Ra recombinante, en particulier l'anakinra, l'autre antagoniste de cytokine est l'antagoniste d'interleukine protéine IL-1Ra naturelle, en particulier l'orthokine, et la composition pharmaceutique convenant pour une administration locale.

4. Trousse contenant une composition pharmaceutique contenant un antagoniste de cytokine et facultativement un facteur de croissance, une composition pharmaceutique contenant un corticoïde, et également un autre antagoniste de cytokine, l'antagoniste de cytokine étant l'antagoniste d'interleukine protéine IL-1Ra recombinante, en particulier l'anakinra, l'autre antagoniste de cytokine étant l'antagoniste d'interleukine protéine IL-1Ra naturelle, en particulier l'orthokine, et la composition pharmaceutique convenant pour une administration locale.

5. Composition pharmaceutique selon la revendication 2 ou 3 ou trousse selon la revendication 4, la ou les compositions pharmaceutiques étant destinées à une administration simultanée ou séquentielle de l'antagoniste de cytokine ou du facteur de croissance et du corticoïde.

6. Composition pharmaceutique ou trousse selon l'une des revendications 1, 2 et 4 à 5, dans laquelle l'antagoniste de cytokine est présent à une concentration de 0,5 à 150 mg/dose dans la composition pharmaceutique contenant l'antagoniste de cytokine.

7. Composition pharmaceutique ou trousse selon l'une des revendications 1 à 6, dans laquelle le corticoïde est
(a) un glucocorticoïde tel que par exemple, cortisone, hydrocortisone, prednisone, prednisolone, cloprednol, déflazacort, fluocortine, triamcinolone, dexaméthasone, méthylprednisolone, fluprednisolone, clocortolone, clobétasone, alclométhasone, fluméthasone, fluprednidène, flurandrénolone, bêtaméthasone, béclométhasone, fluocortolone, mométasone, fluticasone, halométhasone, fluocinolone, diflorasone, désoxyméthasone, fluocinonide, amcinonide, halcinonide, diflucortolone, clobétasol, paraméthasone ;
(b) un minéralocorticoïde tel que par exemple l'aldostérone, la désoxycorticostérone et la fludrocortisone ; ou
(c) un androgène tel que par exemple la déhydroépiandrostérone (DHEA) et des oestrogènes ;
ou un de leurs sels, esters ou médicaments.

8. Composition pharmaceutique ou trousse selon l'une des revendications 1 et 3 à 7, le corticoïde étant présent à une concentration de 1 à 80 mg/dose dans la composition pharmaceutique contenant le corticoïde.

9. Composition pharmaceutique ou trousse selon l'une des revendications 1 à 8, dans laquelle le facteur de croissance est choisi parmi le groupe constitué de TGF-β, IGF, BMP, HGF et VEGF.

10. Composition pharmaceutique ou trousse selon l'une des revendications 1 à 9 pour l'utilisation dans le traitement de maladies articulaires telles que arthrose et perte de cartilage inflammatoire, maladies des tendons et/ou maladies dégénératives de la colonne vertébrale.

11. Composition pharmaceutique ou trousse pour l'utilisation selon la revendication 10, l'arthrose étant une arthrose activée ou une arthrose inflammatoire.

12. Composition pharmaceutique ou trousse pour l'utilisation selon la revendication 10, la maladie dégénérative de la colonne vertébrale étant une hernie discale.

13. Composition pharmaceutique ou trousse selon l'une des revendications 1 à 9 pour l'utilisation dans le traitement de la neurodermite ou de l'alopécie en plaques.

14. Composition pharmaceutique ou trousse selon l'une des revendications 1 à 9, ou composition pharmaceutique ou trousse pour l'utilisation selon l'une des revendications 10 à 13, l'administration locale étant choisie parmi le groupe constitué par une injection dans la région corporelle concernée, en particulier dans l'articulation concernée, dans la racine nerveuse concernée ou dans le disque intervertébral concerné ou dans son environnement local ; une injection intra-articulaire ; et une administration topique.

15. Composition pharmaceutique ou trousse selon l'une des revendications 1 à 9 et 14, ou composition pharmaceutique ou trousse pour l'utilisation selon l'une des revendications 10 à 14, la ou les compositions pharmaceutiques contenant en outre un véhicule et/ou un excipient.

16. Utilisation d'un antagoniste de cytokine, d'un autre antagoniste de cytokine et facultativement d'un facteur de croissance pour la préparation d'une composition pharmaceutique pour l'utilisation dans un traitement combiné conjointement avec un corticoïde, l'antagoniste de cytokine étant l'antagoniste d'interleukine protéine IL-1Ra recombinante, en particulier l'anakinra, l'autre antagoniste de cytokine étant l'antagoniste d'interleukine protéine IL-1Ra naturelle, en particulier l'orthokine, et la composition pharmaceutique convenant pour une administration locale.

17. Utilisation d'un corticoïde pour la préparation d'une composition pharmaceutique pour l'utilisation dans un traitement combiné conjointement avec un antagoniste de cytokine, un autre antagoniste de cytokine et facultativement un facteur de croissance, l'antagoniste de cytokine étant l'antagoniste d'interleukine protéine IL-1Ra recombinante, en particulier l'anakinra, l'autre antagoniste de cytokine étant l'antagoniste d'interleukine protéine IL-1Ra naturelle, en particulier l'orthokine, et la composition pharmaceutique convenant pour une administration locale.
